# FASCICULE DE BREVET EUROPEEN

(11) **EP 1 296 947 B1**
(45) Date de publication et mention de la délivrance du brevet: **04.02.2004**
(21) Numéro de dépôt: 01954058.2
(22) Date de dépôt: 06.07.2001
(51) Int. Cl.: C07D 209/42, A61K 31/475, A61P 9/12

(54) **FORME CRISTALLINE ALPHA DU SEL DE TERT-BUTYLAMINE DU PERINDOPRIL**
KRISTALLFORM ALPHA DES PERINDOPRIL-TERT-BUTYLAMINSALZES
$g(A) CRYSTALLINE FORM OF PERINDOPRIL TERT-BUTYLAMINE SALT

(30) Priorité: 06.07.2000 FR 0008793
(43) Date de publication de la demande: 02.04.2003
(73) Titulaire: Les Laboratoires Servier, 92415 Courbevoie Cedex (FR)
(72) Inventeur: PFEIFFER, Bruno, F-95320 Saint Leu la Forêt (FR); GINOT, Yves-Michel, F-45000 Orléans (FR); COQUEREL, Gérard, F-76520 Boos (FR); BEILLES, Stéphane, F-21000 DIJON (FR)
(86) Numéro de dépôt international: PCT/FR2001/002167
(87) Numéro de publication internationale: WO 2001/087835

(56) Documents cités:
- EP-A- 0 049 658
- EP-A- 0 308 339
- EP-A- 0 308 341

## Description

La présente invention concerne une nouvelle forme cristalline α du sel de tert-butylamine du perindopril de formule (1) : 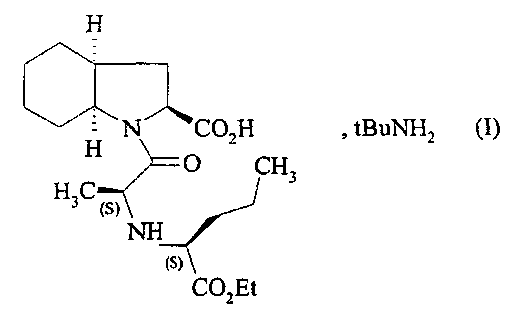 son procédé de préparation ainsi que les compositions pharmaceutiques qui la contiennent.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte tenu de l'intérêt pharmaceutique de ce composé, il était primordial de l'obtenir avec une excellente pureté. Il était également important de pouvoir le synthétiser selon un procédé facilement transposable à l'échelle industrielle, et notamment sous une forme permettant une filtration et un séchage rapides. Enfin, cette forme devait être parfaitement reproductible, facilement formulée et suffisamment stable pour autoriser son stockage prolongé sans conditions particulières de température, de lumière, d'humidité ou de taux d'oxygène.

Le brevet EP 0 308 341 décrit un procédé de synthèse industrielle du perindopril. Cependant, ce document ne précise pas les conditions d'obtention du perindopril sous une forme présentant ces caractéristiques de manière reproductible.

La demanderesse a présentement trouvé qu'un sel particulier du perindopril, le sel de tert-butylamine, pouvait être obtenu sous une forme cristalline bien définie, parfaitement reproductible et présentant notamment des caractéristiques intéressantes de filtration, de séchage et de facilité de formulation.

Plus spécifiquement, la présente invention concerne la forme cristalline α du composé de formule (I), caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, d'intensité et d'intensité relative (exprimée en pourcentage par rapport à la raie la plus intense) :

| **Angle 2 thêta (°)** | **Distance inter-réticulaire d (Å)** | **Intensité** | **Intensité relative (%)** |
|---|---|---|---|
| 7,680 | 11,50 | 390 | 8,8 |
| 8,144 | 10,85 | 230 | 5,2 |
| 9,037 | 9,78 | 4410 | 100 |
| 10,947 | 8,08 | 182 | 4,1 |
| 13,150 | 6,73 | 82 | 1,9 |
| 13,687 | 6,46 | 83 | 1,9 |
| 14,627 | 6,05 | 582 | 13,2 |
| 15,412 | 5,74 | 770 | 17,5 |
| 16,573 | 5,34 | 1115 | 25,3 |
| 17,357 | 5,10 | 340 | 7,7 |
| 18,109 | 4,89 | 193 | 4,4 |
| 19,922 | 4,45 | 306 | 6,9 |
| 20,609 | 4,31 | 375 | 8,5 |
| 21,412 | 4,15 | 226 | 5,1 |
| 21,832 | 4,07 | 217 | 4,9 |
| 22,158 | 4,01 | 483 | 11 |
| 22,588 | 3,93 | 386 | 8,8 |
| 23,323 | 3,81 | 107 | 2,4 |
| 24,200 | 3,67 | 448 | 10,2 |
| 24,727 | 3,60 | 137 | 3,1 |
| 25,957 | 3,43 | 125 | 2,8 |
| 26,932 | 3,31 | 75 | 1,7 |
| 27,836 | 3,20 | 197 | 4,5 |
| 28,966 | 3,08 | 129 | 2,9 |
| 29,213 | 3,05 | 117 | 2,7 |

L'invention s'étend également au procédé de préparation de la forme cristalline α du composé de formule (I), caractérisé en ce que l'on porte à reflux une solution du sel de tert-butylamine du perindopril dans l'acétate d'éthyle, puis on refroidit progressivement jusqu'à cristallisation complète.
- Dans le procédé de cristallisation selon l'invention, on peut utiliser le composé de formule (I) obtenu par n'importe quel procédé. Avantageusement, on utilise le composé de formule (I) obtenu par le procédé de préparation décrit dans le brevet EP 0 308 341.
- La concentration du composé de formule (I) dans l'acétate d'éthyle est préférentiellement comprise entre 70 et 90 g/l.
- Avantageusement, la solution du composé de formule (I) dans l'acétate d'éthyle à reflux est d'abord refroidie jusqu'à une température comprise entre 55 et 65°C à un rythme compris entre 5 et 10°C/h, préférentiellement entre 6 et 8°C/h, puis jusqu'à température ambiante.
- La solution peut être avantageusement ensemencée pendant l'étape de refroidissement à une température comprise entre 76 et 65 °C.
- Le sel de tert-butylamine du perindopril qui est ainsi obtenu se présente sous la forme de bâtonnets individualisés de 0,2 mm de long environ. Cette distribution homogène a pour avantage de permettre une filtration et un séchage particulièrement rapides et efficaces, ainsi que la préparation de formulations pharmaceutiques ayant une composition constante et reproductible, ce qui est particulièrement avantageux lorsque ces formulations sont destinées à l'administration orale.
- La forme ainsi obtenue est suffisamment stable pour autoriser son stockage prolongé sans conditions particulières de température, de lumière, d'humidité ou de taux d'oxygène.

L'invention s'étend aussi aux compositions pharmaceutiques renfermant comme principe actif la forme cristalline α du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les compositions pharmaceutiques selon l'invention peuvent également contenir un diurétique comme l'indapamide.

Les exemples suivants illustrent l'invention mais ne la limitent en aucune façon.

Le spectre de diffraction X sur poudre a été mesuré avec les conditions expérimentales suivantes :
- Diffractomètre Siemens D5005, détecteur à scintillations,
- Anticathode de cuivre (λ=1,5405 Å), voltage 40 KV, intensité 40mA,
- Montage θ-θ,
- Domaine de mesures : 5° à 30°,
- Incrémentation entre chaque mesure : 0,02°,
- Temps de mesure par pas : 2s,
- Fentes variables : v6,
- Filtre Kβ (Ni),
- Pas de référence interne,
- Procédure de zéro avec les fentes Siemens,
- Données expérimentales traitées avec le logiciel EVA (version 5.0).

### EXEMPLE 1 : Forme cristalline α du sel de tert-butylamine du perindopril

125 g du sel de tert-butylamine du perindopril obtenu selon le procédé décrit dans le brevet EP 0 308 341 sont dissous dans 1,68 1 d'acétate d'éthyle portés au reflux.
La température de la solution est ensuite ramenée à 60°C en 2h30, puis refroidie jusqu'à température ambiante.
Le solide obtenu est collecté par filtration.

### Diagramme de diffraction X sur poudre :

Le profil de diffraction des rayons X de la poudre (angles de diffraction) de la forme α du sel de tert-butylamine du perindopril est donné par les raies significatives rassemblées dans le tableau suivant, avec l'intensité et l'intensité relative (exprimée en pourcentage par rapport à la raie la plus intense).

| **Angle 2 thêta (°)** | **Distance inter-réticulaire d (Å)** | **Intensité** | **Intensité relative (%)** |
|---|---|---|---|
| 7,680 | 11,50 | 390 | 8,8 |
| 8,144 | 10,85 | 230 | 5,2 |
| 9,037 | 9,78 | 4410 | 100 |
| 10,947 | 8,08 | 182 | 4,1 |
| 13,150 | 6,73 | 82 | 1,9 |
| 13,687 | 6,46 | 83 | 1,9 |
| 14,627 | 6,05 | 582 | 13,2 |
| 15,412 | 5,74 | 770 | 17,5 |
| 16,573 | 5,34 | 1115 | 25,3 |
| 17,357 | 5,10 | 340 | 7,7 |
| 18,109 | 4,89 | 193 | 4,4 |
| 19,922 | 4,45 | 306 | 6,9 |
| 20,609 | 4,31 | 375 | 8,5 |
| 21,412 | 4,15 | 226 | 5,1 |
| 21,832 | 4,07 | 217 | 4,9 |
| 22,158 | 4,01 | 483 | 11 |
| 22,588 | 3,93 | 386 | 8,8 |
| 23,323 | 3,81 | 107 | 2,4 |
| 24,200 | 3,67 | 448 | 10,2 |
| 24,727 | 3,60 | 137 | 3,1 |
| 25,957 | 3,43 | 125 | 2,8 |
| 26,932 | 3,31 | 75 | 1,7 |
| 27,836 | 3,20 | 197 | 4,5 |
| 28,966 | 3,08 | 129 | 2,9 |
| 29,213 | 3,05 | 117 | 2,7 |

### EXEMPLE 2 : Composition pharmaceutique

Formule de préparation pour 1000 comprimés dosés à 4 mg :

| | |
|---|---|
| Composé de l'exemple 1 | 4 g |
| Hydroxypropylcellulose | 2 g |
| Amidon de blé | 10 g |
| Lactose | 100 g |
| Stéarate de magnésium | 3 g |
| Talc | 3 g |

## Revendications

1. Forme cristalline α du composé de formule (I) : 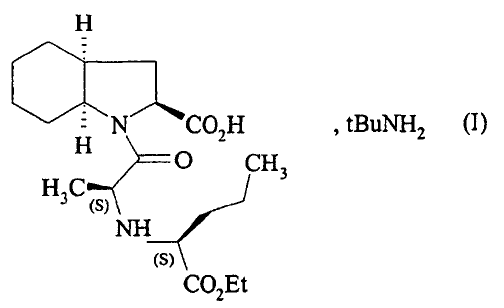 **caractérisée par** le diagramme de diffraction X sur poudre suivant, mesuré sur un diffractomètre (anticathode de cuivre) et exprimé en termes de distances inter-réticulaires d, d'angle de Bragg 2 thêta, d'intensité et d'intensité relative (exprimée en pourcentage par rapport à la raie la plus intense) :
| **Angle 2 thêta (°)** | **Distance inter-réticulaire d (Å)** | **Intensité** | **Intensité relative (%)** |
|---|---|---|---|
| 7,680 | 11,50 | 390 | 8,8 |
| 8,144 | 10,85 | 230 | 5,2 |
| 9,037 | 9,78 | 4410 | 100 |
| 10,947 | 8,08 | 182 | 4,1 |
| 13,150 | 6,73 | 82 | 1,9 |
| 13,687 | 6,46 | 83 | 1,9 |
| 14,627 | 6,05 | 582 | 13,2 |
| 15,412 | 5,74 | 770 | 17,5 |
| 16,573 | 5,34 | 1115 | 25,3 |
| 17,357 | 5,10 | 340 | 7,7 |
| 18,109 | 4,89 | 193 | 4,4 |
| 19,922 | 4,45 | 306 | 6,9 |
| 20,609 | 4,31 | 375 | 8,5 |
| 21,412 | 4,15 | 226 | 5,1 |
| 21,832 | 4,07 | 217 | 4,9 |
| 22,158 | 4,01 | 483 | 11 |
| 22,588 | 3,93 | 386 | 8,8 |
| 23,323 | 3,81 | 107 | 2,4 |
| 24,200 | 3,67 | 448 | 10,2 |
| 24,727 | 3,60 | 137 | 3,1 |
| 25,957 | 3,43 | 125 | 2,8 |
| 26,932 | 3,31 | 75 | 1,7 |
| 27,836 | 3,20 | 197 | 4,5 |
| 28,966 | 3,08 | 129 | 2,9 |
| 29,213 | 3,05 | 117 | 2,7 |

2. Procédé de préparation de la forme cristalline α du composé de formule (I) selon la revendication 1, **caractérisé en ce que** l'on porte à reflux une solution du sel de tert-butylamine du perindopril dans l'acétate d'éthyle, puis on refroidit progressivement jusqu'à cristallisation complète.

3. Procédé selon la revendication 2, **caractérisé en ce que** l'on utilise le composé de formule (I) obtenu par le procédé de préparation décrit dans le brevet EP 0 308 341.

4. Procédé selon l'une quelconque des revendications 2 ou 3, **caractérisé en ce que** la concentration du composé de formule (I) dans l'acétate d'éthyle est comprise entre 70 et 90 g/l.

5. Procédé selon l'une quelconque des revendications 2 à 4, **caractérisé en ce que** la solution du composé de formule (I) dans l'acétate d'éthyle à reflux est d'abord refroidie jusqu'à une température comprise entre 55 et 65°C à un rythme compris entre 5 et 10°C/h, puis jusqu'à température ambiante.

6. Procédé selon l'une quelconque des revendications 2 à 4, **caractérisé en ce que** la solution du composé de formule I dans l'acétate d'éthyle est ensemencée pendant l'étape de refroidissement à une température comprise entre 76 et 65 °C

7. Procédé selon la revendication 5, **caractérisé en ce que** la solution du composé de formule (I) dans l'acétate d'éthyle à reflux est d'abord refroidie jusqu'à une température comprise entre 55 et 65°C à un rythme compris entre 6 et 8°C/h, puis jusqu'à température ambiante.

8. Procédé selon l'une quelconque des revendications 2 à 7, **caractérisé en ce que** le sel de tert-butylamine du perindopril qui est ainsi obtenu se présente sous forme de bâtonnets individualisés facilement filtrables.

9. Composition pharmaceutique contenant comme principe actif le composé selon la revendication 1, en combinaison avec un ou plusieurs véhicules inertes, non toxiques et pharmaceutiquement acceptables.

10. Composition pharmaceutique selon la revendication 9 utile pour la fabrication de médicaments utiles en tant qu'inhibiteur de l'enzyme de conversion de l'angiotensine I.

11. Composition pharmaceutique selon la revendication 10 utile pour la fabrication de médicaments utiles dans le traitement des maladies cardiovasculaires.

12. Composition pharmaceutique selon l'une quelconque des revendications 9 à 11 **caractérisée en ce qu'**elle contient également un diurétique.

13. Composition pharmaceutique selon la revendication 12 **caractérisée en ce que** le diurétique est l'indapamide.

## Patentansprüche

1. α-Kristallform der Verbindung der Formel (I): 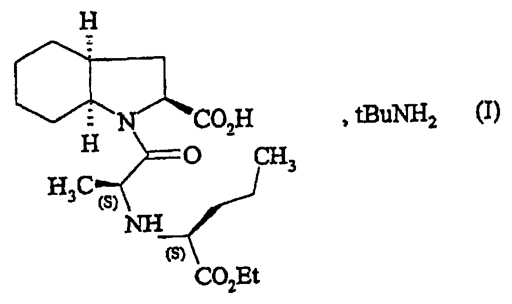 **gekennzeichnet durch** das folgende Pulverröntgenbeugungsdiagramm, welches mit Hilfe eines Diffraktometers (Kupfer-Antikathode) gemessen worden ist und welches als die Gitterebenenabstände d, den Bragg-Winkel 2 Theta, die Intensität und die relative Intensität (ausgedrückt als Prozentsatz bezogen auf die intensivste Bande) angegeben ist:
| **2 Theta-Winkel (°)** | **Gitterebenenabstand d (Å)** | **Intensität** | **Relative Intensität (%)** |
|---|---|---|---|
| 7,680 | 11,50 | 390 | 8,8 |
| 8,144 | 10,85 | 230 | 5,2 |
| 9,037 | 9,78 | 4410 | 100 |
| 10,947 | 8,08 | 182 | 4,1 |
| 13,150 | 6,73 | 82 | 1,9 |
| 13,687 | 6,46 | 83 | 1,9 |
| 14,627 | 6,05 | 582 | 13,2 |
| 15,412 | 5,74 | 770 | 17,5 |
| 16,573 | 5,34 | 1115 | 25,3 |
| 17,357 | 5,10 | 340 | 7,7 |
| 18,109 | 4,89 | 193 | 4,4 |
| 19,922 | 4,45 | 306 | 6,9 |
| 20,609 | 4,31 | 375 | 8,5 |
| 21,412 | 4,15 | 226 | 5,1 |
| 21,832 | 4,07 | 217 | 4,9 |
| 22,158 | 4,01 | 483 | 11 |
| 22,588 | 3,93 | 386 | 8,8 |
| 23,323 | 3,81 | 107 | 2,4 |
| 24,200 | 3,67 | 448 | 10,2 |
| 24,727 | 3,60 | 137 | 3,1 |
| 25,957 | 3,43 | 125 | 2,8 |
| 26,932 | 3,31 | 75 | 1,7 |
| 27,836 | 3,20 | 197 | 4,5 |
| 28,966 | 3,08 | 129 | 2,9 |
| 29,213 | 3,05 | 117 | 2,7 |

2. Verfahren zur Herstellung der α-Kristallform der Verbindung der Formel (I) nach Anspruch 1, **dadurch gekennzeichnet, daß** man eine Lösung des tert.-Butylaminsalzes von Perindopril in Ethylacetat zum Sieden am Rückfluß erhitzt und dann progressiv bis zur vollständigen Kristallisation abkühlt.

3. Verfahren nach Anspruch 2, **dadurch gekennzeichnet, daß** man die Verbindung der Formel (I) verwendet, die man mit Hilfe des in dem Patent EP 0 308 341 beschriebenen Verfahrens erhalten hat.

4. Verfahren nach einem der Ansprüche 2 oder 3, **dadurch gekennzeichnet, daß** die Konzentration der Verbindung der Formel (I) in Ethylacetat zwischen 70 und 90 g/l beträgt.

5. Verfahren nach einem der Ansprüche 2 bis 4, **dadurch gekennzeichnet, daß** die zum Sieden erhitzte Lösung der Verbindung der Formel (I) in Ethylacetat zunächst mit einer Geschwindigkeit zwischen 5 und 10°C/h auf eine Temperatur zwischen 55 und 65°C und dann auf Raumtemperatur abgekühlt wird.

6. Verfahren nach einem der Ansprüche 2 bis 4, **dadurch gekennzeichnet, daß** die Lösung der Verbindung der Formel I in Ethylacetat während der Stufe des Abkühlens auf eine Temperatur zwischen 76 und 65°C angeimpft wird.

7. Verfahren nach Anspruch 5, **dadurch gekennzeichnet, daß** die am Rückfluß gehaltene Verbindung der Formel (I) in Ethylacetat zunächst mit einer Geschwindigkeit zwischen 6 und 8°C/h auf eine Temperatur zwischen 55 und 65°C und dann auf Raumtemperatur abgekühlt wird.

8. Verfahren nach einem der Ansprüche 2 bis 7, **dadurch gekennzeichnet, daß** das in dieser Weise erhaltene tert.-Butylaminsalz von Perindopril in Form von vereinzelten, leicht filtrierbaren Stäbchen vorliegt.

9. Pharmazeutische Zubereitung enthaltend als Wirkstoff die Verbindung nach Anspruch 1 in Kombination mit einem oder mehreren inerten, nichttoxischen und pharmazeutisch annehmbaren Trägermaterialien.

10. Pharmazeutische Zubereitung nach Anspruch 9 für die Herstellung von Arzneimitteln, die als Inhibitoren des Angiotensin I umwandelnden Enzyms nützlich sind.

11. Pharmazeutische Zubereitung nach Anspruch 10 für die Herstellung von Arzneimitteln, die bei der Behandlung von kardiovaskulären Erkrankungen nützlich sind.

12. Pharmazeutische Zubereitung nach einem der Ansprüche 9 bis 11, **dadurch gekennzeichnet, daß** sie zusätzlich ein Diuretikum enthält.

13. Pharmazeutische Zubereitung nach Anspruch 12, **dadurch gekennzeichnet, daß** das Diuretikum Indapamid ist.

## Claims

1. α crystalline form of the compound of formula (I) : 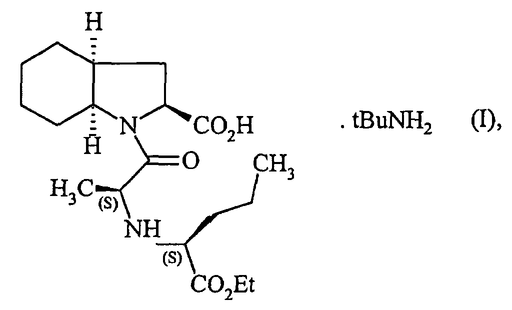 **characterised by** the following powder X-ray diffraction diagram, measured using a diffractometer (copper anticathode) and expressed in terms of inter-planar distances d, Bragg's angle 2 theta, intensity and relative intensity (expressed as a percentage with respect to the most intense ray) :
| **Angle 2 theta (°)** | **Inter-planar distance d (Å)** | **Intensity** | **Relative intensity (%)** |
|---|---|---|---|
| 7.680 | 11.50 | 390 | 8.8 |
| 8.144 | 10.85 | 230 | 5.2 |
| 9.037 | 9.78 | 4410 | 100 |
| 10.947 | 8.08 | 182 | 4.1 |
| 13.150 | 6.73 | 82 | 1.9 |
| 13.687 | 6.46 | 83 | 1.9 |
| 14.627 | 6.05 | 582 | 13.2 |
| 15.412 | 5.74 | 770 | 17.5 |
| 16.573 | 5.34 | 1115 | 25.3 |
| 17.357 | 5.10 | 340 | 7.7 |
| 18.109 | 4.89 | 193 | 4.4 |
| 19.922 | 4.45 | 306 | 6.9 |
| 20.609 | 4.31 | 375 | 8.5 |
| 21.412 | 4.15 | 226 | 5.1 |
| 21.832 | 4.07 | 217 | 4.9 |
| 22.158 | 4.01 | 483 | 11 |
| 22.588 | 3.93 | 386 | 8.8 |
| 23.323 | 3.81 | 107 | 2.4 |
| 24.200 | 3.67 | 448 | 10.2 |
| 24.727 | 3.60 | 137 | 3.1 |
| 25.957 | 3.43 | 125 | 2.8 |
| 26.932 | 3.31 | 75 | 1.7 |
| 27.836 | 3.20 | 197 | 4.5 |
| 28.966 | 3.08 | 129 | 2.9 |
| 29.213 | 3.05 | 117 | 2.7 |

2. Process for the preparation of the α crystalline form of the compound of formula (I) according to claim 1, **characterised in that** a solution of perindopril tert-butylamine salt in ethyl acetate is heated at reflux and is then cooled gradually until crystallisation is complete.

3. Process according to claim 2, **characterised in that** the compound of formula (I) obtained by the preparation process described in patent specification EP 0 308 341 is used.

4. Process according to either claim 2 or claim 3, **characterised in that** the concentration of the compound of formula (I) in the ethyl acetate is from 70 to 90 g/litre.

5. Process according to any one of claims 2 to 4, **characterised in that** the solution of the compound of formula (I) in ethyl acetate at reflux is first cooled to a temperature of from 55 to 65°C at a rate of from 5 to 10°C/hour, and then to ambient temperature.

6. Process according to any one of claims 2 to 4, **characterised in that** the solution of the compound of formula I in ethyl acetate is seeded during the cooling step at a temperature of from 76 to 65°C.

7. Process according to claim 5, **characterised in that** the solution of the compound of formula (I) in ethyl acetate at reflux is first cooled to a temperature of from 55 to 65°C at a rate of from 6 to 8°C/hour, and then to ambient temperature.

8. Process according to any one of claims 2 to 7, **characterised in that** the perindopril tert-butylamine salt that is thereby obtained is in the form of readily filterable individual needles.

9. Pharmaceutical composition comprising as active ingredient the compound according to claim 1, in combination with one or more pharmaceutically acceptable, inert, nontoxic carriers.

10. Pharmaceutical composition according to claim 9 for use in the manufacture of medicaments for use as inhibitors of angiotensin I converting enzyme.

11. Pharmaceutical composition according to claim 10 for use in the manufacture of medicaments for use in the treatment of cardiovascular diseases.

12. Pharmaceutical composition according to any one of claims 9 to 11, **characterised in that** it also comprises a diuretic.

13. Pharmaceutical composition according to claim 12, **characterised in that** the diuretic is indapamide.
